# EUROPEAN PATENT APPLICATION

(11) **EP 1 437 155 A1**
(43) Date of publication of application: **14.07.2004**
(21) Application number: 01274484.3
(22) Date of filing: 15.11.2001
(51) Int. Cl.: A61M 25/06

(54) **CATHETER AND USE METHOD THEREOF**

(30) Priority: 14.09.2001 ES 200102074
(71) Applicant: S.C.B., S.A., 48001 Bilbao (ES)
(72) Inventor: ACHA GANDARIAS, Pedro, 48990 Vizcaya Getxo (ES)
(74) Representative: Primo de Rivera y Urquijo, Jose Antonio
(86) International application number: PCT/ES2001/000438
(87) International publication number: WO 2003/024517

(57) **Abstract**

The catheter is of the type provided with a needle and a cannula for the introduction thereof by means of only one hand into the blood conduits of a living being's body, preferably a human body, and is composed of three independent assembled bodies. The first of them is a hollow cylinder open on its rear end and having a cannula joined to its front end, in addition to guide means assembled with the guide means of a second body or casing. The cannula is open on its upper, front and rear part, and its front end is provided with a needle joined to it with semicircular section along the majority of its length. A perforated and hollow plunger is introduced inside of the cylinder along its entire length, with a flap and pusher on its upper part, its lower end being provided with a connection.

## Description

### OBJECT OF THE INVENTION

The catheter and use method consist of the use of a device composed of three main parts, cylinder with cannula, plunger and casing with needle, which permit the use of the invention by means of only one hand, that is, it achieves introducing the needle and the cannula in the desired place (blood vessel, hollow viscus, etc.) on the first try by using only one hand, and assuring the hygiene of the operation without there being contact between the atmosphere and the internal device components, preventing infections or the transmission of contaminants as well as the introduction of air in the conduits or bodies.

This device and use method thereof have a special application in the medical or veterinary sector for the intravenous introduction of fluid products in a living being's body, or preferably in the human body, as well as for extracting blood or any other body fluid, drug administration, etc.

### DESCRIPTION OF THE STATE OF THE ART

There are a multitude of catheters and similar devices in the state of the art for introducing needles into a living being's body with the smallest risk for the being and for the purpose of supplying fluid elements in the vein, mainly serum.

US patent US 4,950,252, belonging to Luther Medical Products, Inc., discloses a coupling of a needle, catheter and a device for protecting the needle by means of a body inside of which the needle slides. Said body is held with only one hand and a cannula is fixed thereto, inside of which the needle slides, which is also joined to the body. With only one hand, the needle and cannula are introduced into the body, without being able to see if both have been introduced in the vein or, if, on the contrary, a second puncture will be necessary in order to introduce the elements into the vein. Once introduced in the body and without ensuring the correct introduction in the vein, the needle is withdrawn from the inside of the cannula and from the body, a flap existing in the upper part of the body and which is joined to the needle, shifting backwards. After having withdrawn the needle and if the cannula was correctly introduced, the latter is separated from the body, and the infusion equipment in turn connected to the recipient with the fluid to be supplied is coupled to the free end of the cannula.

The main problem of this device, like with any of those existing on the market, is that it needs contact with the outside, the atmosphere, in the moment in which the cannula is to be coupled to the infusion equipment, which can result in a dangerous entrance of air into the veins or in the contamination of the blood flow in contact with the atmosphere. Another resulting drawback is that in the moment of the puncture, that is, when inserting the needle and cannula, it is impossible to determine if the cannula is in the desired place or not, possibly having passed over it, which would imply carrying out a new puncture.

Another device similar to the aforementioned one is disclosed in the also US Patent 5,456,668, filed by F.H. Faulding & Co. Limited, and consists of a system in which the needle is hidden inside of a body after having been introduced, together with the cannula, into the body. This device has the same drawbacks as the previously described device.

Another novel feature of the device of the present invention is the needle used for introducing the cannula into the vein, and which permits the subsequent needle withdrawal. A needle prior to the one disclosed in this patent specification is disclosed in US patent 4,354,491, belonging to Steven L. Marbry, in which a continuous groove is seen along the entire needle length, inside of which a cannula is introduced, said needle being subsequently withdrawn through a widening thereof in the end opposite to that of the introduction into the body. The configuration of the needle prevents the cannula from moving from its place, inside of the needle, until the user so wishes, but by means of the configuration of the present invention, the same function is filled with simpler and more advantageous design and features.

With regard to the use process currently used, it basically consists of introducing a hollow needle inside of a plastic cannula and is provided with a transparent or translucent body, like a deposit, on the opposite end. Once the needle with the cannula is introduced in the desired place, it is checked if it was correctly introduced in said place. This checking is carried out by seeing if the rear transparent or translucent body of the catheter has filled with blood and if so, the cannula slides through the needle inside of the puncture area, subsequently withdrawing the needle and leaving the cannula inside of the body in order to subsequently connect the final end of the catheter to an infusion equipment connected to a recipient with fluid content.

This device has the previously disclosed drawbacks referring to the impossibility of a correct placement of the needle tip inside of the body, since, even though the rear catheter body contains blood or fluid, this does not mean the permanency of the needle tip in the desired place of the body. When the aforementioned occurs, the plastic cannula does not slide inside of the body with the consequential failure of the maneuver and repetition thereof, causing hemorrhages, hematomas, vein rupture, infections, etc.

Another drawback that the previous devices have is that referring to the lack of hygiene and possibility of contamination and subsequent infection, and the entrance of air (this may cause an air embolism), when, in the case that the cannula is correctly introduced into the body, the needle is withdrawn and the rear part of the plastic cannula and the blood on the inside thereof are in contact with the outside contaminating means, air.

### DESCRIPTION

The present invention consists of coupling these three independent elements or bodies forming a catheter for the purpose of introducing the cannula into the desired place (blood vessel, hollow viscus, etc.), ensuring its correct placement for the subsequent supply of a fluid component or an extraction of fluid from the inside.

The independent components or bodies are a cylinder with the cannula to be introduced in the patient joined thereto, a plunger shifting along the length of the aforementioned cylinder, subsequently coupled to a plastic tube with a connection for an infusion equipment, and a cylindrical casing with the needle joined to it, on which the cylinder will shift linearly. The cylinder is introduced in the carcass and will shift along the length of it through the guides arranged on the outside of the cylinder assembled on the tracks of the inside of the carcass. The plunger is located inside of the cylinder and on its end can have a plastic tube coupled to the infusion equipment and subsequently to the supply recipient. The cannula joined to the cylinder is located inside of the needle, the latter horizontally sectioned along its diameter except in the front part in which the walls can be raised above the horizontal cut, like a curve hump, the sectioned curve surface continuing.

Having assembled the three components forming the catheter, the latter is ready for use with only one hand, holding it on one side with the thumb and the index finger above, on the flap joined to the plunger, and holding the side opposite to the one of the thumb with the remaining fingers. Prior to its use, it is possible to purge all the elements already connected to the recipient fluid. To use it, the needle-cannula assembly is introduced in the desired area, and to check if the cannula and needle are really introduced in the body, it slightly aspirates with the plunger pushed by the flap on which the index finger acts, such that if it is in the desired place, the cylinder will fill with blood or liquid from the body. Once we are sure that it is in the suitable place, with no need to repeat punctures with the subsequent pain for the patient and deterioration of the puncturing body, due to the "in vein placement detection" system, the flap is pushed with the same index finger, inducing the consequent shifting of the cannula inside of the body. Once the cannula is introduced in the body, the casing with the needle is removed from the inside thereof as a result of its grooved opening permitting separation of the cannula and needle, only the cannula remaining inside of the vein and the cylinder and plunger supported outside on the patient's arm.

By opening the shutoff cock between the supply recipient and the rear end of the plunger, the necessary fluid component is thereby provided to the patient, with no danger of the entrance of air inside of the device and with no risk of infections due to contact with the atmosphere; since the system is purged from the beginning of the operation.

### DESCRIPTION OF THE DRAWINGS

In order to facilitate the understanding of the present invention, 19 figures are attached to the present application whose purpose is to better understand the principles on which the invention concerning us is based and the better understanding of the description of a preferred embodiment form, taking into account that the character of the figures is illustrative and non-limiting.
Figure 1 shows an exploded perspective view of the catheter components and a detail of two possible forms of the free end of the needle.

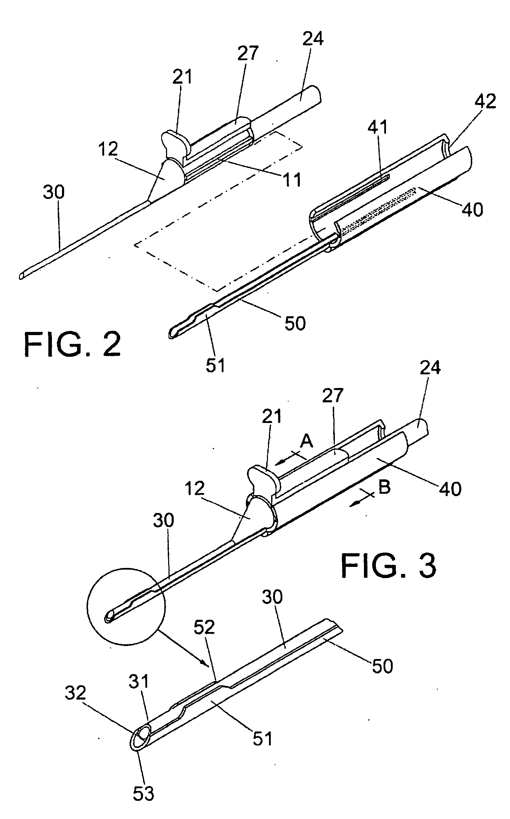

Figure 2 shows a perspective view of the catheter prior to the assembly of two of its components.
Figure 3 shows a perspective view of the catheter prepared to be used and a detail of the needle.

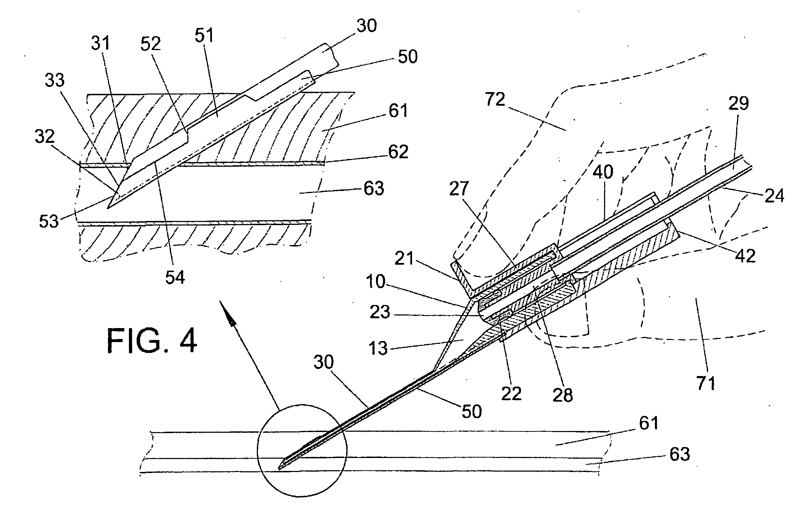

Figure 4 shows a cross section view of the catheter when introduced in the body of a patient and an enlarged detail of the introduction of the needle/cannula into the vein.

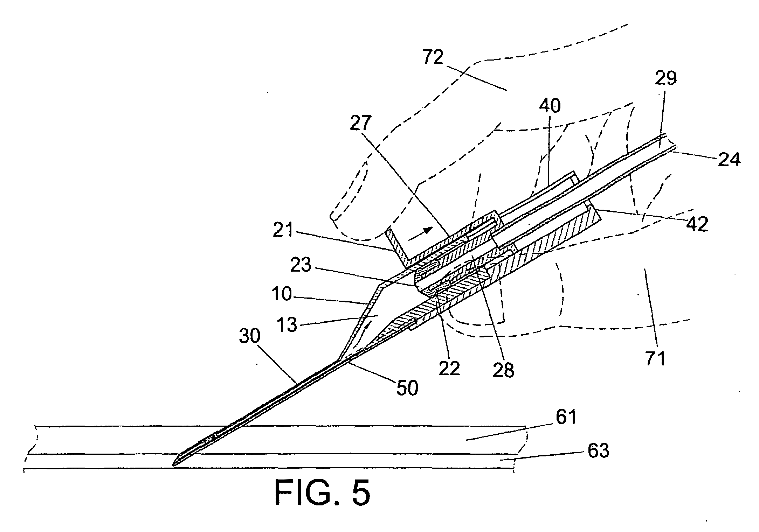

Figure 5 shows a cross section view of the catheter with the cannula and needle introduced in the vein and aspirating blood.

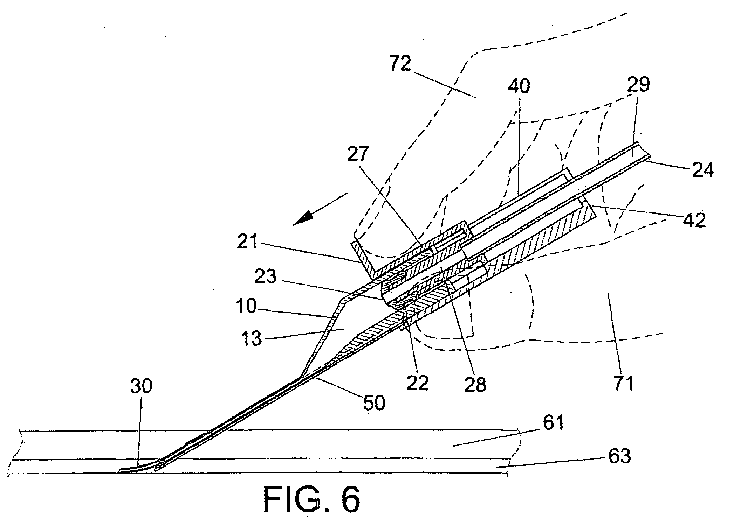

Figure 6 shows a cross section view of the catheter with the cannula being introduced into the vein.

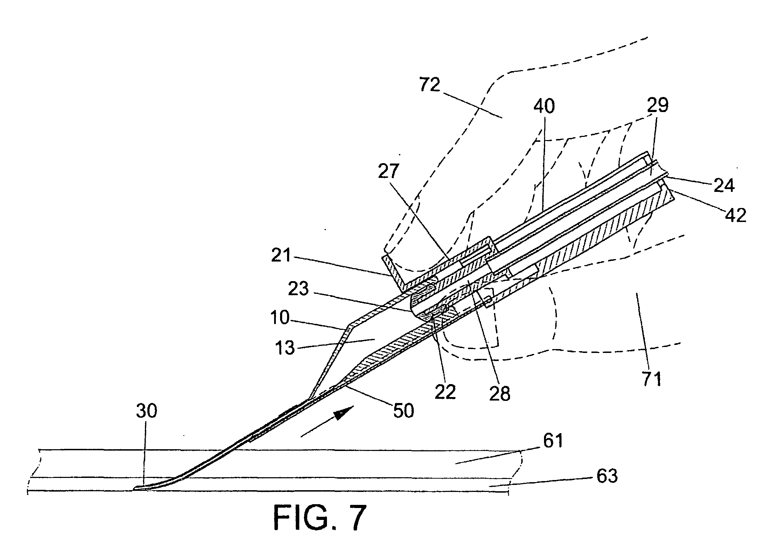

Figure 7 shows a cross section view of the catheter with the cannula introduced in the vein and the needle being withdrawn.

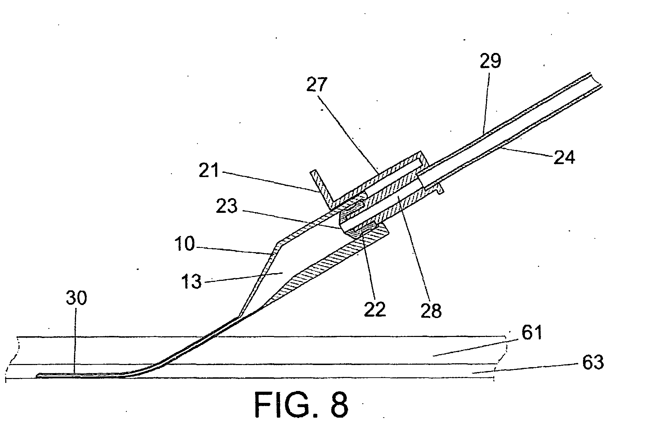

Figure 8 shows a cross section view of the catheter with the needle already withdrawn and the cannula perfectly introduced in the vein.

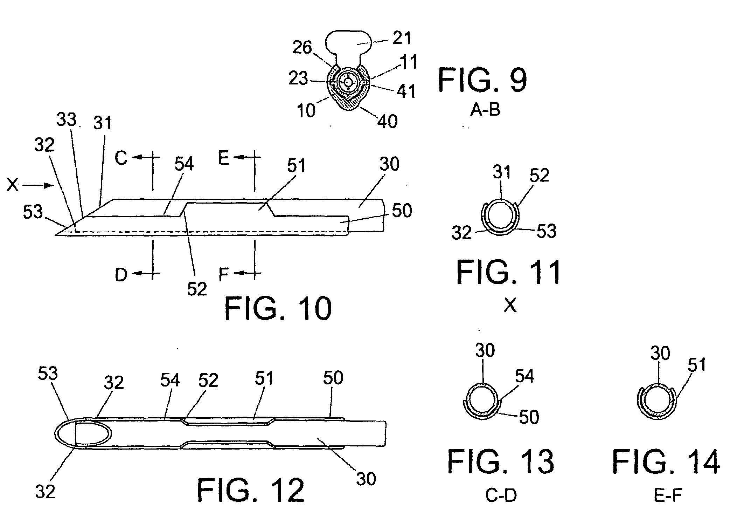

Figure 9 shows a section after cutting the A-B plane indicated in figure 3.
Figure 10 shows an enlargement of the needle with the cannula.
Figure 11 shows a front view of the elements in figure 10.
Figure 12 shows a plan view of the needle with the cannula.
Figure 13 shows a section after cutting the C-D plane indicated in figure 10.
Figure 14 shows a section after cutting the E-F plane indicated in figure 10.

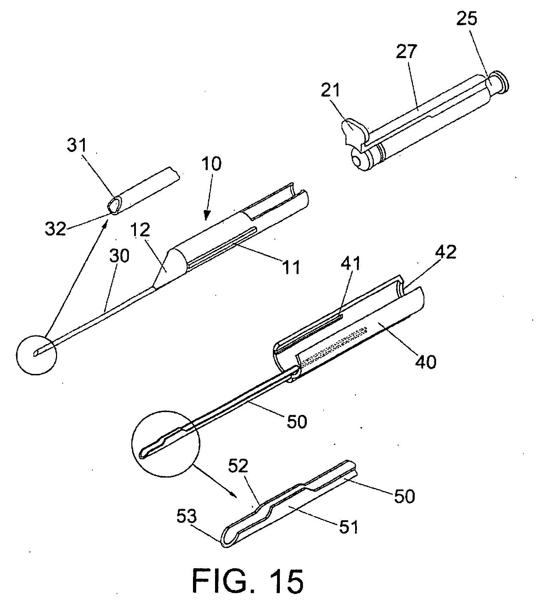

Figure 15 shows an exploded perspective view of a second preferred catheter form.

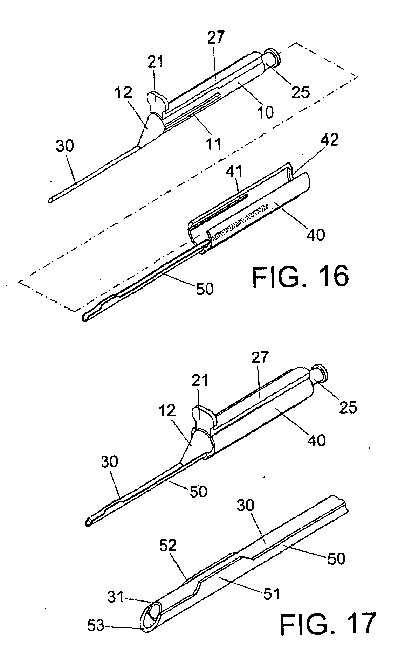

Figure 16 shows a perspective view of the second preferred catheter form prior to the assembly of two of its components.
Figure 17 shows a perspective view of the second preferred catheter form prepared to be used and a detail of the needle.

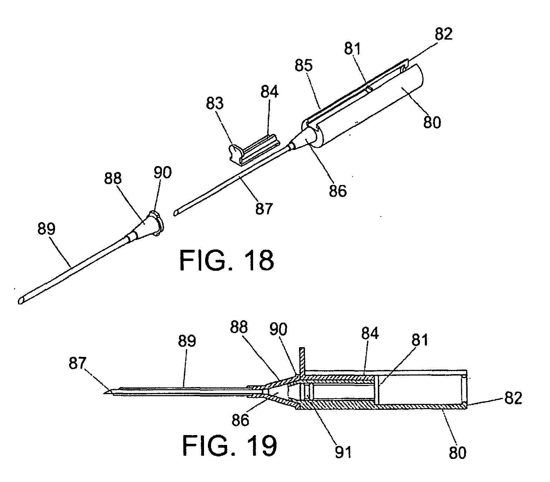

Figure 18 shows another preferred catheter form in which the needle is introduced inside of the cannula.
Figure 19 shows a cross section view of the catheter with the needle introduced in the cannula.

### DESCRIPTION OF A PREFERRED EMBODIMENT

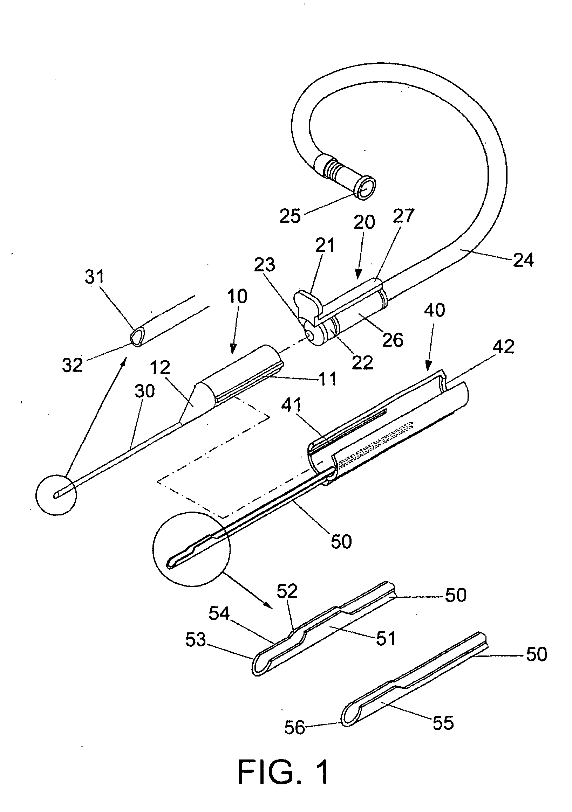

In figure 1, all the components of the catheter object of the present invention can be seen in a situation prior to their assembly. Before assembling the catheter, it has three independent bodies, a cylinder 10 with the cannula 30 to be introduced in the patient joined to the cylinder 10, a plunger ,20 introduced on the inside of the cylinder 10, creating a hermetically sealed compartment due to the rubber 22 on the end of the plunger 20 and which shifts along the length the cylinder 10, and a cylindrical casing 40 with the needle 50 joined to it, on which the cylinder 10 and cannula 30 will linearly shift.

In turn, the cylinder 10, hollow inside and open on its rear end, is provided with three differentiated parts starting from its front inclined end 12 where the cannula 30 is joined to the cylinder 10 and the inside of the cannula 30, also hollow, is joined to the hollow inside of,the cylinder 10. Said cylinder is provided with a track 11 on each one of its two sides, by which the guides 41 made on the sides of the inside of the casing 40 slide. The front end of the cannula 30, opposite to the point joined to the cylinder 10, is provided with two crossed planes defining the shape of its free end, the cannula having its upper section 31 inclined in one direction and the lower section 32 inclined in another direction, thus coinciding with the inclination of the needle 50 and not surpassing the latter.

The plunger 20 which is introduced and slides inside of the cylinder 10 is perforated by means of a cavity 23 crossing through it completely, and its front end is provided with an also hollow rubber plug 22. Between the main cylindrical body 26 of the plunger 20 and the pusher 27 coupled to the plunger by its rear end and intended so that the plunger 20 be shifted along the length of the cylinder 10 with the aid of a flap 21 placed on the front end and perpendicular to the pusher 27, there is an empty or hollow space so that the cylinder 10 can be introduced and thus the plunger 20 remains housed inside of the cylinder 10. The rear part, the plunger 20 is provided with a flexible conduit 24 for the purpose of carrying fluid inside of the patient. This can be coupled through the connector 25 at the end of the conduit 24 to an infusion equipment or to another fluid connected to a recipient containing a fluid component, such as serum.

The third main body, the casing 40, is provided with, in addition to the guides 41 for housing the tracks 11 of the cylinder 10, a cylindrical body open on its upper part, as well as in its front and rear parts, and a rim or projection 42 in the rear part so as to prevent the plunger 20 from coming out of the cylinder 10 in one of the catheter handling movements, specifically in the backwards shifting. The front side of this casing 40 has a needle 50 joined to it sectioned in half in the majority of its length, with two possible structures: the first structure has a hump 51 on its end close the end of the needle 50, almost converting the semicircular section of the needle 50 into circular with an inclination 52 towards the front part of the needle which is followed by a horizontal span 54 in order to end in a tip 53 with an inclination; in the second structure, the inclined part 56 of the hump 55 coincides with the free end or tip 56 of the needle 50.

The catheter assembly is very simple and consists of introducing the plunger 20 into the inside of the cylinder 10 and subsequently making the tracks 11 of the outside of the cylinder 10 coincide with the guides 41 on the inside of the casing. Having introduced the cylinder 10 into the inside of the casing 40, it is seen how the cannula 30, joined to the cylinder 10, remains within the space limited by the needle 50 and by the hump 51 of the needle 50, the inclination of the upper section 31 of the cannula 30 coinciding on the free end with the inclination of the free end of the needle 53. The hump 51 thus ensures that the cannula 30 does not shift vertically with regard to the needle 50.

The use method of the catheter is suitable for being used with only one hand with no risk of contamination, infection or the entrance of air and it has been designed for this purpose. If desired, it is possible to first purge all the material, to do so, the serum bottle is connected to the infusion equipment and to the connector 25, after this, the infusion equipment shutoff cock is turned on in order to thus purge all the material. In order to hold the catheter, the thumb 71 is placed on a side of the casing 40, the index finger 72 is supported on the rear part of the flap 21 and on the sliding element 27, and the remaining fingers on the side opposite to the thumb support holding the casing 40. Once the device is fixed, it is tilted for the purpose of introducing the cannula 30, supported on the needle 50, into the patient. This begins by introducing the tip of the needle 50, whose inclination is the same as the inclination of the upper part of the cannula 31, both inclined elements thereby coinciding in a span 33 in order to thus facilitate the introduction thereof into the patient. After introducing the needle 50 and the cannula 30 such that these pass through the skin and subcutaneous tissue 61, it reaches the wall of the vein 62, the place where the cannula 30 must be introduced. To do so, it crosses said wall of the vein 62 by slightly introducing the tip 53 of the needle and the front end 31 of the cannula inside of the vein 63. Once this front part is introduced in the vein 63, it must be checked if the cannula 30 is inside of the vein 63, since it could happen that it completely passes through it with the subsequent erroneous introduction of the cannula 30 outside of the vein 63, ensuring the subsequent correct and complete introduction of the cannula 30 in the vein 63 by means of said check.

In order to carry out said check once the components are in the previously indicated location, the index finger 72 is supported on the front part of the flap 21, instead of behind it, and the finger 72 is removed, such that the sliding element 27 shifts backwards, which causes the corresponding suction of blood from inside the vein 63, blood which, after passing through the inside of the cannula 30, enters in the chamber 13 of the cylinder 10, which is defined by the front inclined part 12 of the cylinder. Due to the translucent features of the materials used in the cylinder and the plunger, the user can check if the chamber 13 has been filled with blood and verifies that the free end of the needle 50 and of the cannula 30 are inside of the vein 63, that is, in the correct position for the complete introduction of the cannula 30.

In order to continue introducing the cannula 30, the position of the index finger 72 is varied again, the latter again being supported on the rear part of the flap 21 and on the sliding element 27, and upon pushing the flap 21 forwards (towards the vein 63), it causes the cannula 30 to slide in the direction of the vein 63 with regard to the needle 50 is caused, as well as that of the entire "cylinder-plunger" 10-20 assembly, with regard to the casing 40 on the guides 41 and tracks 11.

Once the cannula 30 has been introduced into the inside of the vein 63, the needle 50 is withdrawn from the inside thereof 63, to do so, the casing 40 is pulled backwards, but carefully so as to ensure that the cannula 30 remains inside of the vein 63. The front end of the needle 50, with its curved elevation 51 so as to prevent the cannula 30 from being diverted, releases the latter 30 by means of a slight deformation of the cannula 30, of a flexible material, projecting through the groove existing between the two elevated curved walls 51 of the needle 50.

Once the cannula 30 is introduced into the vein 63 and the "cylinder-plunger" 10-20 assembly secured to the patient's body, the shutoff cock (not shown) located on the infusion equipment connected to the conduit 24 is turned on, such that the fluid from the bag, for example serum, is introduced into the patient's body. In order to prevent the patient from handling the plunger when it is secured to his body, it is possible to separate the flap 21 from the sliding element 27 by means of slight manual pressure.

A variant of the previous device consists of providing a longer, plunger, with the coupling nexus 25 directly incorporated at the end thereof and not through a conduit 24. The arrangement of the remaining components is identical to the previously detailed assembly.

Another variant of the previous examples, for the purpose of using the needles 87 and cannulae 89 of the catheters currently used, such that it is not necessary to manufacture elements with a particular design, consists of the use of needles 87 introduced inside of the cannula 89. The functioning is similar to that described, but unlike the currently used device, in which it is not necessary to suction in order to check that the cannula is in place, in this device it is possible to aspirate the fluid or blood with the plunger in order to verify or check that the cannula 89 is correctly placed inside of the vein 63.

For this, the device is provided with a cylindrical body 80 with several guides 85 on the upper part thereof intended for housing the pusher body, formed by a body with tracks on its sides 84 and a flap 83. Inside of the cylindrical body 80 there is a plunger 91 defined on its rear part by a wall 81 on which the sliding element 84 is supported. The cylindrical body 80 is provided with a rim 82 on its rear end for preventing the plunger 91 from coming out of the cylinder 80. The rear part of the cylinder 80 is provided with a needle 87 joined to the cylinder through a conical body 86. This needle 87 is placed inside of the cannula 89, the latter being fixed to the cylindrical body 80 due to the introduction of the conical body 88 of the cannula 89 on the conical body of the cylinder 80.

The functioning varies from the previous one in that once the cannula 89 and needle 87 are introduced inside of the vein and after checking the correct placement thereof by aspirating blood when the flap 83 is pulled, it is necessary to subsequently withdraw the needle and the cylindrical body completely, which forces the cannula to be opened to the outside environment through its conical body 88 with the subsequent danger of contamination for the patient. In order to reduce this danger, it is necessary to join a conduit as soon as possible to the end of the cannula 90 for introducing the serum, for example, in the patient's body.

The invention, within its essentiality, can be carried out in practice in other embodiment forms differing only in details from the one indicated only as an example. This invention can therefore be carried out in any shape and size, with the most suitable means and materials and with the most convenient accessories, the component elements possibly being replaced with other technically equivalent ones, for all this to be comprised within the claims.

## Claims

1. A catheter, of the type provided with a needle (50) and a cannula (30) for the introduction thereof by means of only one hand into a living being's blood conduits, preferably a human body, **characterized in that** it comprises three independently assembled bodies, these bodies being:
- a hollow cylinder (10) open on its rear end, with a cannula (30) joined to its front end and guide means (11);
- a perforated and hollow plunger (20) along its entire length, with a flap (21) and pusher (27) on its upper part, its rear end being provided with a plastic tube (24) provided with a connection (25),
- and optionally a casing (40) with guide means (41) and open on its upper, front and rear part, its front end provided with a needle (50) joined to it with semicircular section along the majority of its length,
such that for said components to be assembled, the plunger (20) is introduced inside of the cylinder (10), assembling the latter to the casing (40) by means of the guide means (11, 41) and the cannula (30) being introduced into the needle (50).

2. A catheter according to claim 1, **characterized in that** the plunger (20) is provided with a flap (21) perpendicular to a pusher (27) joined to the front end thereof, said pusher (27) being joined to the end of the plunger (20) by its rear part, leaving a cavity or empty space between the curved upper surface of the plunger (20) and the lower side of the pusher (27) so that the wall of the cylinder (10) can be introduced into said cavity, the plunger (20) being thus housed inside of the cylinder (10) and placing the pusher (27) and the flap (21) on the outside of the cylinder (10).

3. A catheter according to claim 1, **characterized in that** the front part of the plunger (20) is provided with a perforated (23) rubber plug (22) providing hermetically sealed conditions to the cylinder (10).

4. A catheter according to claim 1, **characterized in that** the flap (21) can be withdrawn from the pusher (27) by means of slight manual pressure.

5. A catheter according to claim 1, **characterized in that** the guide means (11, 41) are composed of a system of guides (41) and tracks (11).

6. A catheter according to claim 1, **characterized in that** the casing (40) is provided with a rim or projection (42) in its open rear end which limits the backwards shifting of the plunger (20).

7. A catheter according to claim 1, **characterized in that** connection (25) system can be placed at the end of a tube (24), said tube (24) being the continuation of the perforation of the plunger (20).

8. A catheter according to claim 1, **characterized in that** the needle (50) is provided with a span in the front part thereof where the needle section is not semicircular, having a curved wall (51) elevated up to almost completing a circular section of the needle and provided with inclined planes constituting the elevation and descent (52) from the semicircular section until the almost circular section of the needle (51), there being after the plane (52) constituting the descent a horizontal span (54) ending on the free inclined end (53) of the needle (50).

9. A catheter according to claim 1, **characterized in that** the needle (50) is provided with a span in the front part thereof, where the needle section is not semicircular, the latter having a curved wall (51) elevated up to almost completing a circular section on the free end of the needle, provided with inclined planes constituting the elevation and descent (56) from the semicircular section until the almost circular section of the needle, the descent plane inclination (56) coinciding with the inclination of the free end (56) of the needle (50).

10. A catheter according to claim 1, **characterized in that** the free end of the cannula (30) is provided with crossed planes (31, 32) defining the shape of its free end, its upper section (31) having the same inclination as the free end (53, 56) of the needle (50) and the lower section (32) inclined in another direction, preventing the cannula (30) from surpassing the needle (50).

11. A method for using a catheter according to the previous claims, **characterized in that** it comprises the following steps:
- assembling the three components (10, 20, 40),
- purging the device prior to use thereof,
- correct manual support of the catheter,
- inserting the needle (50) and the cannula (30) in the desired place of the body (63),
- checking that both components (30, 50) are inside of the desired place of the body (63),
- introducing the cannula (30) into the desired place of the body (63),
- withdrawing the needle (50) and the casing (40).

12. A method for using a catheter according to claim 11, **characterized in that** the user must hold the catheter by supporting the thumb (71) on a side thereof, the index finger (72), according to the operation to be carried out, in front of or behind the flap (21) of the plunger (20), and the remaining fingers on the side opposite to the thumb (71) placement.

13. A method for using a catheter according to claim 11, **characterized in that** the user must shift the flap (21) of the pusher (27), located on the plunger (20), backwards with the index finger (72) in order to aspirate the fluid and to check that the cannula (30) is in the desired place of the body (63).

14. A method for using a catheter according to claim 11, **characterized in that** in order to introduce the cannula (30) in the desired place, the user must push the flap (21) of the plunger (20) forwards with his index finger (72), transmitting movement to the cylinder (10) and the cannula (30) which shifts with regard to the casing (40) and the needle (50), through the guide means (11, 41).

15. A method for using a catheter according to previous claims, **characterized in that** the needle (50) and the casing (40) are withdrawn once the cannula (30) has slid inside of the desired place of the body (63).

16. A catheter according to claim 1, **characterized in that** the plunger and the cylinder are joined in the same body (80), the front part of said body (80) being provided with a needle (87) joined instead of a cannula.

17. A catheter according to claim 16, **characterized in that** it comprises two assembled bodies:
- a cylindrical body (80) and several guides (85) on the upper part thereof intended for housing a,
- pusher body, formed by a flat body with tracks (84) on its sides and a perpendicular flap (83), and
- a cannula (89) inside of which the needle (87) is introduced arranged on the front part of the cylinder (80).

18. A method according to claim 11 for extracting blood in a human or animal body.
